# EUROPEAN PATENT APPLICATION

(11) **EP 1 103 238 A1**
(43) Date of publication of application: **30.05.2001**
(21) Application number: 00650194.4
(22) Date of filing: 16.11.2000
(51) Int. Cl.: A61F 9/007

(54) **Torsional ultrasound handpiece**

(30) Priority: 29.11.1999 US 450800
(71) Applicant: Alcon Universal, Ltd., 6331 Hünenberg (CH)
(72) Inventor: Boukhny, Mikhail, Laguna Beach, CA 92651 (US)
(74) Representative: Hanna, Peter William Derek

(57) **Abstract**

A handpiece (10) suitable for use in ophthalmic phacoemulsification surgery is provided with an ultrasonically driven cutting tip (16), comprising an ultrasound horn (18) having longitudinally vibrating piezoelectric elements. To obtain oscillatory or torsional movement as well as longitudinal movement, a series of generally parallel, circumferentially disposed, diagonal slits (20) is provided in the ultrasonic horn. The slits translate longitudinal vibrations into oscillatory or torsional vibrations.

## Description

### Background of the Invention

This invention relates to ultrasonic devices and more particularly to an ophthalmic phacoemulsification handpiece.

A typical ultrasonic surgical device suitable for ophthalmic procedures consists of an ultrasonically driven handpiece, an attached hollow cutting tip, an irrigating sleeve and an electronic control console. The handpiece assembly is attached to the control console by an electric cable and flexible tubings. Through the electric cable, the console varies the power level transmitted by the handpiece to the attached cutting tip and the flexible tubings supply irrigation fluid to and draw aspiration fluid from the eye through the handpiece assembly.

The operative part of the handpiece is a centrally located, hollow resonating bar or horn directly attached to a set of piezoelectric crystals. The crystals supply the required ultrasonic vibration needed to drive both the horn and the attached cutting tip during phacoemulsification and are controlled by the console. The crystal/horn assembly is suspended within the hollow body or shell of the handpiece at its nodal points by relatively inflexible mountings. The handpiece body terminates in a reduced diameter portion or nosecone at the body's distal end. The nosecone is externally threaded to accept the irrigation sleeve. Likewise, the horn bore is internally threaded at its distal end to receive the external threads of the cutting tip. The irrigation sleeve also has an internally threaded bore that is screwed onto the external threads of the nosecone. The cutting tip is adjusted so that the tip projects only a predetermined amount past the open end of the irrigating sleeve.

When used to perform phacoemulsification, the ends of the cutting tip and irrigating sleeve are inserted into a small incision of predetermined width in the cornea, sclera, or other location in the eye tissue in order to gain access to the anterior chamber of the eye. The cutting tip is ultrasonically vibrated along its longitudinal axis within the irrigating sleeve by the crystal-driven ultrasonic horn, thereby emulsifying upon contact the selected tissue in situ. The hollow bore of the cutting tip communicates with the bore in the horn that in turn communicates with the aspiration line from the handpiece to the console. A reduced pressure or vacuum source in the console draws or aspirates the emulsified tissue from the eye through the open end of the cutting tip, the bore of the cutting tip, the horn bore, and the aspiration line and into a collection device. The aspiration of emulsified tissue is aided by a saline flushing solution or irrigant that is injected into the surgical site through the small annular gap between the inside surface of the irrigating sleeve and the outside surface of the cutting tip.

There have been prior attempts to combine ultrasonic longitudinal motion of the cutting tip with rotational or oscillating motion of the tip, see U.S. Patent Nos. 5,222,959 (Anis), 5,722,945 (Anis, et al.) and 4,504,264 (Kelman). These prior attempts have used electric motors to provide the rotation of the tip which require O-ring or other seals that can fail in addition to the added complexity and possible failure of the motors.

Accordingly, a need continues to exist for a reliable ultrasonic handpiece that will oscillate.

### Brief Summary of the Invention

The present invention improves upon prior art ultrasonic devices by providing a handpiece having a set of longitudinally vibrating piezoelectric elements. To obtain oscillatory or torsional movement, a series of parallel, diagonal slits is provided circumferentially around the ultrasonic horn. The slits translate longitudinal vibrations into oscillatory or torsional vibrations.

It is accordingly an object of the present invention to provide an ultrasound handpiece having both longitudinal and torsional motion.

It is a further object of the present invention to provide an ultrasound handpiece with a horn having a series of diagonal slits to produce torsional motion. Other objects, features and advantages of the present invention will become apparent with reference to the drawings, and the following description of the drawings and claims.

### Brief Description of the Drawings

FIG. 1 is an elevational view of the ultrasonic horn that may be used with the handpiece of the present invention.

FIG. 2 is a cross-section view of the ultrasonic horn that may be used with the handpiece of the present invention taken at line 2-2 in FIG. 1.

FIG. 3 is a perspective view of the ultrasonic horn that may be used with the handpiece of the present invention.

FIG. 4 is a perspective view of the handpiece of the present invention.

FIG. 5 a block diagram of a first driving circuit that may be used with the present invention.

FIG. 6 is a block diagram of a second driving circuit that may be used with the present invention.

### Detailed Description of the Invention

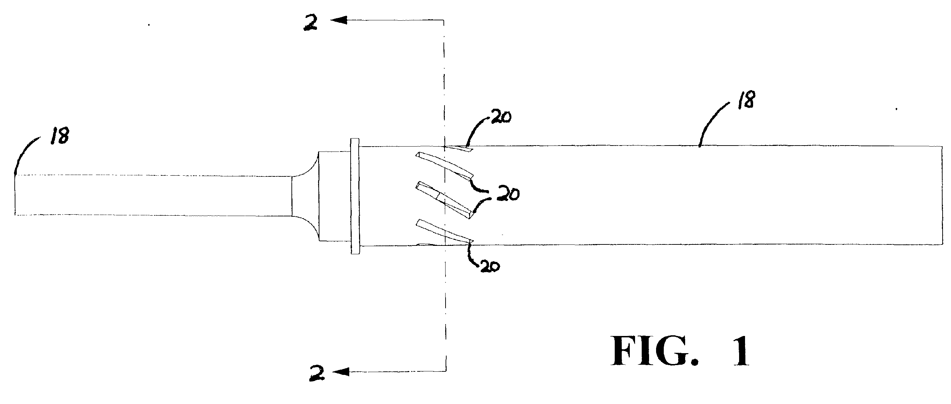

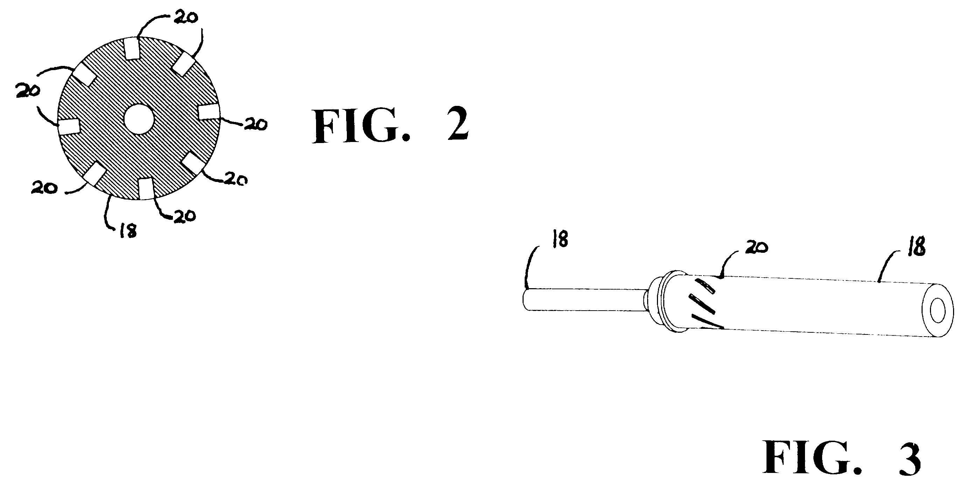

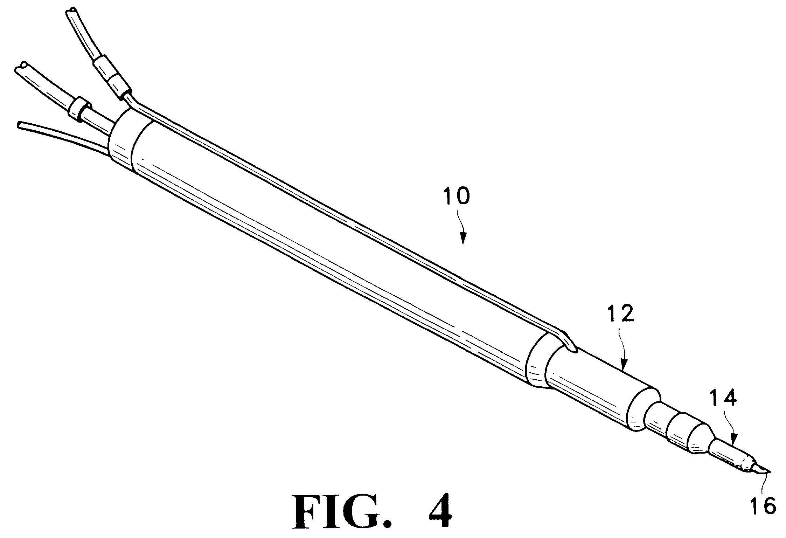

As best seen in FIG. 4, handpiece 10 of the present invention generally includes handpiece shell 12, infusion sleeve 14 and cutting tip 16. As best seen in FIGS. 1-3, handpiece 10 contains ultrasonic hom 18. Handpiece 10 generally contain at least one pair of piezoelectric crystals (not shown) coupled to horn 18 so as to create longitudinal vibratory motion in horn 18 when the crystals are excited. The materials used for and the construction of handpiece 10 and horn 18 (except as to slits 20) are well-known to those skilled in the art. See, for example, U.S. Patent No. 4,169,984 (Parisi), 4,515,583 (Sorich), 4,989,588 (Kubota, et al.) and 5,359,996 (Hood). Horn 18 contains a series of generally parallel, circumferential, diagonal slits 20 near distal end 22 of horn 18. The angle of slits 20 preferably is around 45 degrees to the longitudinal axis of horn 18, which is the principal stress angle for horn 18, but any angle between 10 degrees and 80 degrees may be used, with between 45 degrees and 60 degrees being preferred. As one skilled in the art will recognize, the exact location, number and size of slits 20 needed to convert the longitudinal motion of the crystals to torsional or oscillatory motion of distal end 22 of horn 18 will vary depending upon the amount of torsional motion desired. Generally speaking, efficient conversion will occur with slits 20 of between 0.001 inches and 0.4 inches deep. In addition, slits 20 need not be parallel to each other.

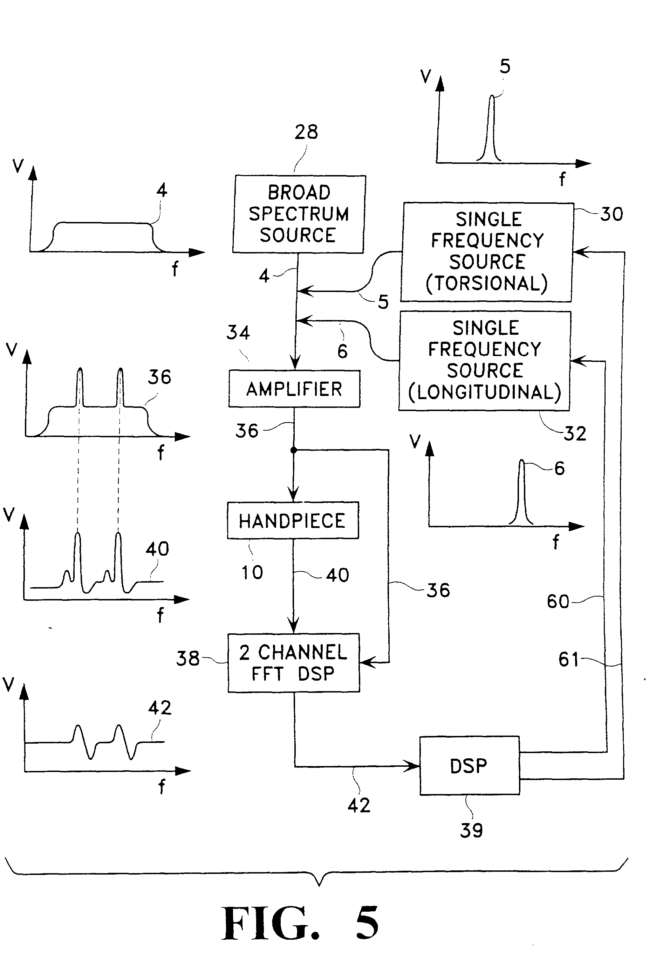

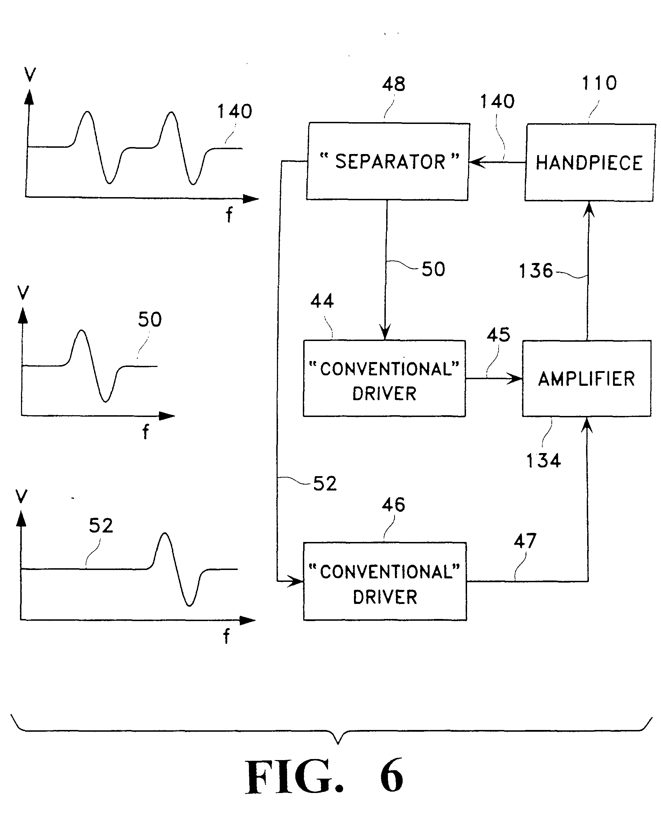

As seen in FIG. 5, ultrasound generator 26 employs a broad-spectrum source to generate at least a component of the signal that drives an ultrasonic handpiece ("the drive signal"). The broad-spectrum source may be programmable and thus easily adjustable by varying certain input information fed to the source. However, a fixed-spectrum source may also be used without difficulty. A fast fourier transform ("FFT") digital signal processor ("DSP") may be used to analyze the response of handpiece 10 to the broad-spectrum component of the drive signal. In real-time applications, the output of the FFT DSP is used to generate control parameters embodied within an appropriate feedback signal, which is fed to the circuitry generating the drive signal in order to alter aspects of the drive signal. As seen in FIG. 6, ultrasound generator 26 may also use a conventional signal processor to analyze the response of handpiece 10 to the drive signal. The term "drive signal" as used here encompasses at least a signal useful solely for powering an ultrasonic handpiece, a signal useful solely for tuning or calibrating a handpiece, and a combination of such a power signal and such a tuning or calibration signal.

As shown in FIG. 5, broad spectrum signal source 28 generates drive signal 4 which is combined with drive signals 5 and 6 from torsional single frequency source 30 and longitudinal single frequency source 32, respectively, in amplifier 34. Amplifier 34 delivers drive signal 36 to handpiece 10 and to FFT DSP 38. FFT DSP 38 also receives feedback signal 40 from handpiece 10. FFT DSP 38 processes drive signal 36 and feedback signal 40 in the manner more fully disclosed in commonly owned U.S. Patent Application Serial No. 08/769,257 (corresponding to PCT Patent Application No. PCT/US97/15952), to determine the operating characteristics of handpiece 10. FFT DSP 38 determines the electrical response of handpiece 10 on broad spectrum signal 4 and provides signal 42 to DSP 39 which generates adjusting signals 60 and 61 to adjust the frequencies and/or output voltage of sources 32 and 30, respectively so as to adjust drive signals 5 and 6.

As shown in FIG. 6, two conventional drive signal sources, such as those described in U.S. Patent No. 5,431,664, or U.S. Patent Application Serial No. 08/769,257 (corresponding to PCT Patent Application No. PCT/US97/15952), may be used. For example, source 44 may generate drive signal 45 for torsional crystals 18 and source 46 may generate driving signal 47 for longitudinal crystals 20. Drive signals 45 and 47 are combined in amplifier 134 and drive signal 136 delivered to handpiece 110. Handpiece feedback signal 140 is filtered through separator 48 to provide adjusting signals 50 and 52 to sources 44 and 46. Separator 48 may be any number of commercially available analog or digital devices such low pass or high pass filters or heterodyne receiver.

While certain embodiments of the present invention have been described above, these descriptions are given for purposes of illustration and explanation. Variations, changes, modifications and departures from the systems and methods disclosed above may be adopted without departure from the scope of the present invention.

## Claims

1. An ultrasonic surgical device comprising an ultrasonically driven handpiece (10) having a handpiece shell (12), an ultrasound horn (18) held within the shell, an attached hollow cutting tip (16), wherein the handpiece is adapted to be attached to an electronic control console, and in which the cutting tip is arranged for both longitudinal and oscillatorary movement,
characterized in that the horn (18) is provided with a plurality of circumferentially disposed, diagonally arranged slits (20) sized and spaced so as to produce oscillatorary or torsional movement in the horn in response to longitudinal vibration of the horn.

2. The ultrasonic surgical device of claim 1, in which the slits (20) are generally parallel to each other.

3. The ultrasonic surgical device of claim 1 or claim 2, in which the horn (18) has a longitudinal axis and the slits (20) are arranged at an angle of between 10 degrees and 80 degrees to the longitudinal axis.

4. The ultrasonic surgical device of claim 3, in which the slits (20) are arranged at an angle of between 45 degrees and 60 degrees to the longitudinal axis.

5. The ultrasonic surgical device of any one of claims 1 to 4, in which the slits (20) are located near to the distal end of the horn (18).
